# DEMANDE DE BREVET EUROPEEN

(11) **EP 4 382 905 A1**
(43) Date de publication de la demande: **12.06.2024**
(21) Numéro de dépôt: 23215630.7
(22) Date de dépôt: 11.12.2023
(51) Int. Cl.: G01N 33/00, G01N 21/3563, G01N 21/359, A01G 7/00

(54) **IDENTIFICATION D ESPÈCES D ARBRES PAR SPECTROMÉTRIE PROCHE INFRAROUGE**

(30) Priorité: 09.12.2022 FR 2213048
(71) Demandeur: Tonnellerie Radoux, 17500 Jonzac (FR); Institut National de la Recherche pour l'Agriculture, l'Alimentation et l'Environnement, 75007 Paris (FR); Office National des Forêts, 94704 Maison-Alfort (FR)
(72) Inventeur: BELMOKHTAR, Nassim, 75007 Paris (FR); BOIZOT, Nathalie, 75007 Paris (FR); CHARPENTIER, Jean-Paul, 75007 Paris (FR); GIORDANENGO, Thomas, 17500 Jonzac (FR); MUSCH, Brigitte, 75012 Paris (FR); LE-GUÉRROUÉ, Bénédicte, 75012 Paris (FR)
(74) Mandataire: Ipsilon

(57) **Abrégé**

L'invention concerne un procédé (100) d'identification d'une espèce d'arbre caractérisé en ce qu'il comprend les étapes suivantes :
- étape 1 (110) : appliquer contre un arbre (1) entier un rayonnement proche infrarouge incident (10) au moyen d'un spectromètre infrarouge (6),
- étape 2 (120) : recevoir, au moyen dudit spectromètre, un rayonnement réfléchi (14) émis par des molécules organiques (12) présentes dans ledit arbre (1),
- étape 3 (130) : traiter le rayonnement réfléchi (14) pour obtenir un spectre proche infrarouge correspondant audit arbre,
- étape 4 (140) : identifier à quelle espèce appartient ledit arbre entier à partir du spectre infrarouge obtenu à l'étape 3 et de spectres proche infrarouge de référence correspondant à des espèces connues.

## Description

### Domaine technique

La présente invention concerne un procédé de détermination d'espèces d'arbres par spectrométrie infrarouge.

### Arrière-plan technologique

La qualité du bois se définie en fonction de l'usage qui en est attendu. Chaque espèce d'arbre possède un bois ayant des caractéristiques spécifiques comme, par exemple, sa composition, ses propriétés anatomiques, ses propriétés physiques ou encore sa durabilité naturelle. Dans le cas particulier du chêne (genre *Quercus),* les différentes espèces n'ont pas la même composition chimique, ni la même richesse en composés extractibles. Les différentes espèces ne sont donc pas toutes propices aux mêmes usages.

Au cours de leur élaboration, les vins et spiritueux sont traditionnellement élevés et vieillis en fûts. Le bois qui constitut le fût confère des caractéristiques olfactives et gustatives spécifiques aux vins et aux spiritueux et le bois de chêne est généralement utilisé pour la fabrication des fûts.

Différents composés sont extraits du fût vers le vin ou le spiritueux. Des molécules volatiles en modifient l'arôme, comme les whiskylactones (arôme de bois frais, de noix de coco), l'eugénol et l'isoeugénol (arôme de clou de girofle), la vanilline (arôme de vanille de pâtisserie) ou encore les aldéhydes furaniques (arôme d'amande, amande grillé). Des tanins hydrolysables sont également extraits dans le vin. Ces tanins ont une incidence directe sur la perception en bouche du vin (astringence, amertume), ainsi que sur les réactions qui se déroulent au cours du vieillissement. Les fûts sont généralement fabriqués en chêne et la qualité sensorielle du vin et des spiritueux n'est pas la même selon l'espèce de chêne utilisée. Il est ainsi intéressant de pouvoir choisir l'espèce du chêne pour fabriquer le fût afin de maîtriser les caractéristiques sensorielles du vin et des spiritueux qui vont y être stockés.

Une méthode pouvant être utilisée pour déterminer quelle est l'espèce d'un arbre est l'analyse anatomique des tissus du bois de cet arbre. Cependant, cette méthode peut être complexe et incertaine et ne permet pas de différencier les différentes espèces de chênes, et ce, même pour un homme de l'art.

Une autre méthode est l'analyse génétique des arbres qui est très fiable et qui a permis, dans le cas du chêne, d'identifier le chêne sessile *Quercus petraea. (Matt.) Liebl.,* le chêne pédonculé *Quercus robur L.,* le chêne pubescent *Quercus pubecens Willd.* et le chêne tauzin *Quercus pyrenaica Willd.* à partir de l'analyse de l'ADN. Cependant, cette méthode nécessite un prélèvement de matériel végétal en forêt ou l'abattage de l'arbre afin de pouvoir procéder à l'analyse. Par ailleurs, le délai de mise en oeuvre de cette analyse génétique est relativement long et les coûts sont conséquents.

Plus récemment, une méthode d'identification de l'espèce d'un arbre basée sur la spectrométrie proche infrarouge a été développée. Cette méthode optique se base sur l'absorption sélective du rayonnement proche infrarouge par les composés organiques de l'arbre. Cette méthode est indirecte et il est nécessaire de faire un étalonnage du spectromètre à l'aide d'une collection d'échantillons de chênes dont l'espèce est connue. La méthode a été testée en conditions de laboratoire (hygrométrie de l'air, température, humidité du bois maîtrisées) afin de discriminer chêne blanc d'Amérique *(Quercus alba L.)* et chêne rouge d'Amérique *(Quercus rubra L.)* à partir de spectres proche infrarouge acquis directement sur morceaux de bois massifs.

Cependant, l'analyse proche infrarouge de laboratoire présente également des inconvénients. En effet, il est nécessaire d'avoir abattu l'arbre et préparé un échantillon pour déterminer son espèce. Par ailleurs, le délai de réalisation de l'analyse reste long car les échantillons doivent être transportés au laboratoire, ce qui ne permet pas une prise de décision rapide directement sur le terrain. Les forestiers peuvent donc difficilement employer ces méthodes afin de réaliser un inventaire de la ressource disponible en forêt. Enfin, il n'existe pas d'outils permettant de discriminer rapidement l'espèce de chêne et de préparer des lots de bois homogènes pour l'industrie.

Il existe donc un besoin de développer une méthode permettant d'identifier l'espèce d'un chêne directement en forêt ou en milieu industriel, qui soit fiable, rapide, peu coûteuse et non destructive pour l'arbre analysé.

### Résumé de l'invention

La présente invention a pour premier objet un procédé d'identification d'une espèce d'arbre caractérisé en ce qu'il comprend les étapes suivantes :
- étape 1 : appliquer contre un arbre entier un rayonnement proche infrarouge incident au moyen d'un spectromètre infrarouge,
- étape 2 : recevoir, au moyen dudit spectromètre, un rayonnement réfléchi émis par des molécules organiques présentes dans ledit arbre,
- étape 3 : traiter le rayonnement réfléchi pour obtenir un spectre proche infrarouge correspondant audit arbre,
- étape 4 : identifier à quelle espèce appartient ledit arbre entier à partir du spectre infrarouge obtenu à l'étape 3 et de spectres proche infrarouge de référence correspondant à des espèces connues.

Ce procédé étant appliqué sur un arbre entier, il est rapide et facile à mettre en oeuvre et ne nécessite pas le prélèvement puis la préparation d'un échantillon de l'arbre. Le procédé représente ainsi une méthode d'identification de l'espèce à laquelle appartient l'arbre qui est non destructive. Le procédé peut ainsi être mis en oeuvre sur l'arbre entier et à l'endroit où il se trouve.

Dans l'invention, un arbre entier est défini comme un arbre vivant ou un arbre abattu stocké sous forme de grume. L'invention est appliquée de préférence à un arbre vivant. Par arbre vivant, on entend un arbre fixé par ses racines dans le sol, un arbre enraciné.

Le rayonnement incident, qui est un ensemble de radiations, peut être appliqué contre différentes parties de l'arbre, notamment contre le bois de coeur appelé duramen, l'aubier ou l'écorce.

Le rayonnement proche infrarouge (connu sous l'anglicisme « near infrared » ou NIR) incident est le rayonnement émis par le spectromètre infrarouge et qui pénètre dans l'arbre. La gamme de fréquence du rayonnement proche infrarouge incident va de 700 nm à 2500 nm, et plus préférentiellement de 900 nm à 1600 nm. Dans la suite de la description, le rayonnement proche infrarouge utilisé sera appelé simplement rayonnement infrarouge ou rayonnement IR.

Le spectromètre infrarouge, et notamment l'émetteur du spectromètre infrarouge, est positionné contre l'arbre, ou encore en contact direct avec une partie de l'arbre pouvant être la surface ou l'intérieur. Le rayonnement IR incident émis contre l'arbre pénètre alors à l'intérieur de l'arbre, et en particulier à quelques millimètres de profondeur par rapport à la surface contre laquelle le rayonnement incident est émis.

Le rayonnement IR émis agit sur les molécules présentes à l'intérieur de l'arbre, et notamment sur les liaisons chimiques des molécules organiques présentes dans l'arbre. Les molécules organiques absorbent alors une partie du rayonnement IR incident et émettent un rayonnement réfléchi qui est caractéristique de la structure de ces molécules, et donc de certaines propriétés du bois de l'arbre ou encore de l'espèce à laquelle appartient l'arbre.

Le rayonnement réfléchi par les molécules est alors reçu par un détecteur présent dans le spectromètre IR et est ensuite traité pour obtenir les données reçues sous forme d'un spectre infrarouge qui est caractéristique des molécules présentes dans l'arbre étudié, le spectre infrarouge correspondant à l'absorbance en fonction du nombre d'ondes. Le spectre infrarouge obtenu correspond à l'empreinte spectrale de l'arbre.

L'arbre peut être tout type d'arbre et peut par exemple être du genre chêne, acacia, châtaignier, ou autre. Pour chaque genre, plusieurs espèces d'arbre existent et l'invention porte sur l'identification des différentes espèces d'arbre pour un genre donné. Par exemple, la méthode selon l'invention est adaptée à l'identification des espèces dans la famille des querkus. Selon un mode de réalisation préféré, l'arbre est un chêne et la méthode selon l'invention peut permettre d'identifier différentes espèces du chêne et notamment les chênes sessiles, chênes pédonculés, chênes pubescents, chênes tauzins, chênes blancs d'Amérique, chênes de Mongolie, ou un de leurs hybrides. Selon un mode encore plus préféré, la méthode de l'invention peut permettre d'identifier une espèce de chêne choisie parmi les chênes sessiles, pédonculés, pubescents et tauzins.

Le tronc d'un arbre comporte une partie interne recouverte d'une écorce. Cette partie interne est formée par deux types de tissus, le duramen également appelé 'bois de coeur' et qui est entouré par l'aubier. L'aubier et le duramen possèdent une composition chimique différente. Selon un mode de réalisation possible, le rayonnement incident est appliqué sur l'écorce de l'arbre. Selon un mode de réalisation préféré, le rayonnement incident est appliqué contre la partie interne de l'arbre. Selon ce mode de réalisation préféré, le rayonnement incident peut être appliqué sur l'aubier ou sur le duramen.

Selon un mode de réalisation préféré, le procédé d'identification peut comporter préalablement à l'étape 1, les étapes suivantes :
étape a : appliquer sur une pluralité d'arbres entiers de référence dont l'espèce est connue un rayonnement proche infrarouge incident au moyen d'un spectromètre proche infrarouge,
étape b : recevoir, pour chaque arbre et au moyen dudit spectromètre, un rayonnement réfléchi émis par des molécules organiques présentes dans ladite pluralité d'arbres de référence sous l'effet du rayonnement infrarouge incident,
étape c : traiter, pour chaque arbre, le rayonnement réfléchi pour obtenir une pluralité de spectres proche infrarouge de référence,
étape d : attribuer chaque spectre proche infrarouge obtenu à l'espèce connue de l'arbre correspondant, et
étape e : développer un modèle d'étalonnage permettant d'identifier l'espèce d'un arbre à partir du spectre infrarouge dudit arbre.

Selon ce mode de réalisation préféré, l'étape 4 d'identifier à quelle espèce appartient l'arbre entier est réalisée en utilisant l'étalonnage développé.

L'espèce à laquelle appartiennent les arbres de référence peut par exemple avoir été déterminée par analyse génétique.

Le modèle d'étalonnage peut être développé en utilisant par exemple des méthodes de classification non supervisées (classification ascendante hiérarchiques, partitionnement K-means), ou supervisées (analyse factorielle discriminante, random forest, support vector machine, discrimination par plus proches voisins). Cependant tout autre méthode de classification permettant d'obtenir un étalonnage peut être utilisée. Selon un mode préféré, une méthode de discrimination supervisée linéaire de régression des moindres carrés partiels (connu sous l'anglicisme partial least squares discriminant analysis ou PLS-DA) est utilisée.

Selon des modes particuliers de réalisation pris seuls ou en combinaison :
- le procédé peut comporter en outre l'étape d'enlever une portion de l'écorce de l'arbre de manière à découvrir une surface de la partie interne de l'arbre, et d'émettre le rayonnement infrarouge sur ladite surface ; de cette manière, il est possible d'accéder à la partie interne de l'arbre qui est une partie plus riche en molécules organiques d'intérêt et qui permettent de discriminer les différentes espèces d'arbre de manière optimisée ; selon ce mode de réalisation, le rayonnement incident peut être appliqué juste sous l'écorce pour analyser l'aubier ; avantageusement, cette étape du procédé est non destructive pour l'arbre analysé ;
- le procédé peut comporter en outre l'étape d'effectuer un carottage de manière à découvrir une zone du coeur de l'arbre, et d'émettre le rayonnement infrarouge sur ladite zone ; de cette manière, il est possible d'émettre le rayonnement infrarouge dans le duramen, au moyen par exemple d'une fibre optique ; avantageusement, cette étape du procédé est non destructive pour l'arbre analysé ;
- le procédé peut comporter une étape 3' de traitement du spectre infrarouge obtenu à l'étape 3, ladite étape 3' comportant l'application d'au moins une méthode permettant de diminuer le bruit et d'améliorer la qualité du spectre ; il est ainsi possible d'appliquer au moins une méthode choisie parmi la normalisation, la correction de ligne de base, le lissage, la dérivation, ou une de leurs combinaisons ; cette étape 3' permet d'améliorer la qualité du spectre infrarouge et donc des informations qu'il apporte concernant l'arbre ;
- les étapes 1, 2 et 3 peuvent être réitérées une ou plusieurs fois de manière à obtenir un ou plusieurs spectres proche infrarouges qui sont ensuite combinés pour obtenir un spectre proche infrarouge moyen de l'arbre, ledit spectre infrarouge moyen étant alors utilisé pour effectuer l'étape 4 de manière à identifier l'espèce de l'arbre entier ; l'étape 4 peut être effectuée de façon automatique au moyen de l'étalonnage préalablement développé ;
- le spectromètre infrarouge utilisé peut être un spectromètre portable ; il est ainsi possible de transporter facilement le spectromètre pour effectuer des mesures en forêt par exemple ; le spectromètre utilisé peut être par exemple le spectromètre commercialisé par la société VIAVI^{®} sous la référence MicroNIR, le spectromètre commercialisé par la société Ocean Insight^{®} sous la référence NIRQuest ou le spectromètre commercialisé par la société Malvern Panalytical^{®} sous la référence ASD ; de préférence le spectromètre utilisé est le MicroNIR ; et
- l'arbre entier peut être un chêne sessile, un chêne pédonculé, un chêne pubescent, un chêne tauzin ou un de leurs hybrides.

La présente invention a pour deuxième objet l'utilisation du procédé selon le premier objet pour déterminer la proportion de différentes espèces d'arbres dans un peuplement forestier. Cette utilisation peut servir à la simple étude du peuplement forestier ou peut également permettre de déterminer l'espèce des arbres abattus ou à abattre, et ainsi de pouvoir réaliser des lots d'arbres de même espèce pour la commercialisation.

La méthode de la présente invention peut également servir en merranderie et en tonnellerie pour trier des grumes et les classer en fonction de leur espèce et donc en fonction des propriétés sensorielles qu'ils confèreront aux vins et spiritueux lorsqu'ils seront transformés en fûts.

### Brève description des figures

La description qui va suivre en regard des dessins annexés, donnés à titre d'exemples non limitatifs, fera bien comprendre en quoi consiste l'invention et comment elle peut être réalisée. Sur les figures annexées :
[fig. 1a-c] Les figures 1a-c représentent l'application d'un spectromètre contre un chêne vivant;
[fig. 2a-b] Les figures 2a-b représentent un procédé d'identification de l'espèce à laquelle appartient l'arbre des figures 1a-c selon un mode de réalisation de l'invention ;
[fig. 3] La figure 3 illustre de manière schématique la mise au point d'un outil de discrimination d'espèces d'arbres ; et
[fig. 4] La figure 4 illustre de manière schématique l'application d'un spectromètre contre un chêne vivant.

### Description de mode(s) de réalisation

Dans les figures, et à moins qu'il n'en soit disposé autrement, les éléments identiques porteront les mêmes signes de référence.

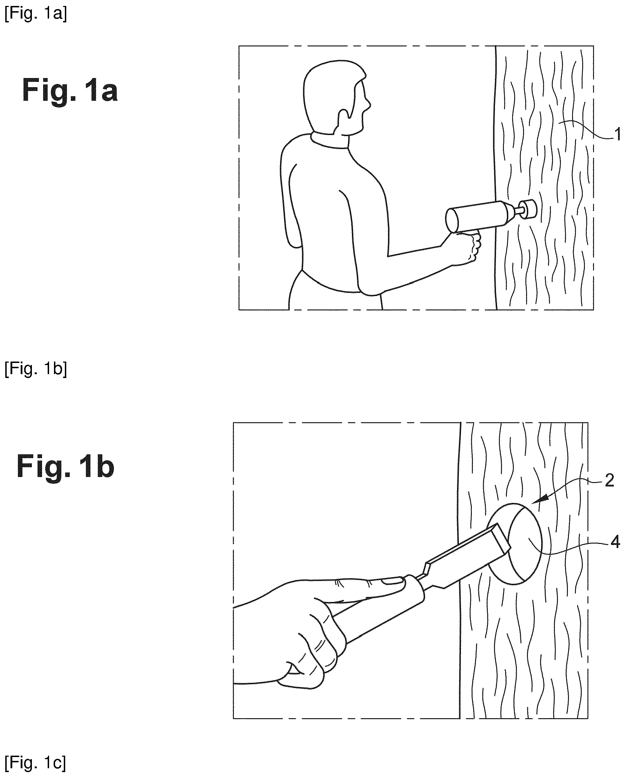

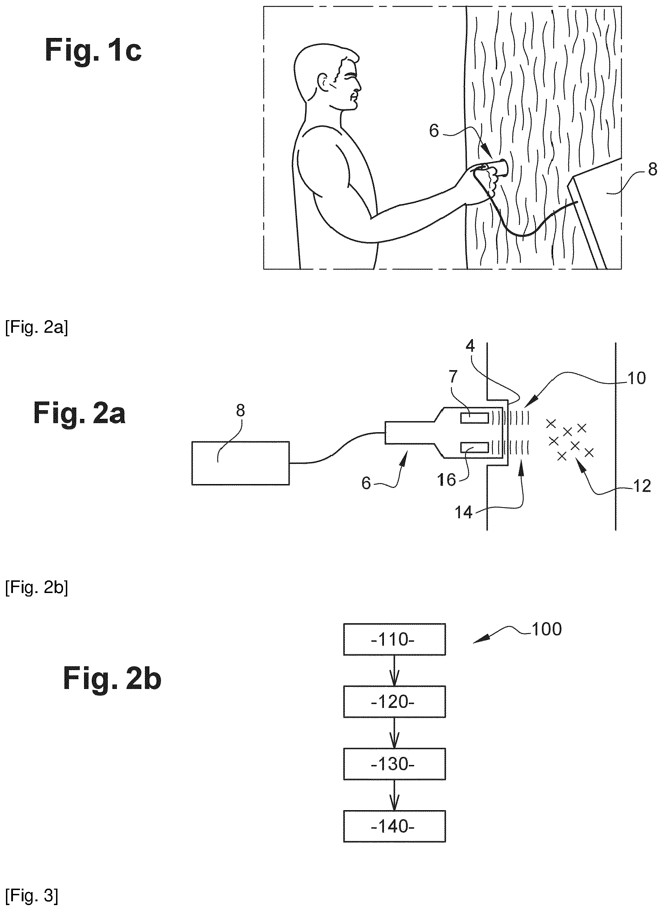

Dans un exemple de réalisation illustré sur les figures 1a à 1c, le procédé est mis en oeuvre dans une forêt de la région Centre-Val de Loire sur des espèces de chêne et le procédé est mis en oeuvre pour identifier à quelle espèce appartient un chêne 1.

Dans cet exemple de réalisation, un morceau d'écorce est enlevé au moyen d'une scie cloche de façon à faire un trou 2 découvrant une surface 4 de la partie interne de l'arbre. Dans ce mode de réalisation, le procédé est mis en oeuvre pour déterminer le spectre proche infrarouge de l'aubier.

Un spectromètre 6 commercialisé par la société Viavi^{®} sous la référence MicroNiR est utilisé et est appliqué contre la surface 4. Le spectromètre 6 est relié à une unité centrale 8 qui permet d'obtenir le spectre proche infrarouge de l'arbre 1, encore appelé empreinte spectrale.

Comme illustré sur les figures 2a et 2b, un procédé 100 selon un exemple de réalisation de l'invention est mis en oeuvre sur l'arbre 1.

Le procédé comporte une première étape 110 dans laquelle un rayonnement proche IR incident 10 est émis au moyen du spectromètre 6, et notamment d'une lampe 7, et pénètre à l'intérieur de l'arbre 1. Des molécules 12 qui sont caractéristiques de l'espèce de l'arbre 1 absorbent une partie du rayonnement IR incident 10 ce qui modifie l'état de vibration de leurs liaisons. Les molécules 12 diffusent ensuite un rayonnement réfléchi 14.

Dans une deuxième étape 120, le rayonnement réfléchi 14 est reçu par un détecteur 16 du spectromètre 6. Le rayonnement réfléchi 14 est alors traité dans une troisième étape 130 par une unité centrale 8 pour obtenir un spectre infrarouge correspondant au rayonnement réfléchi 14.

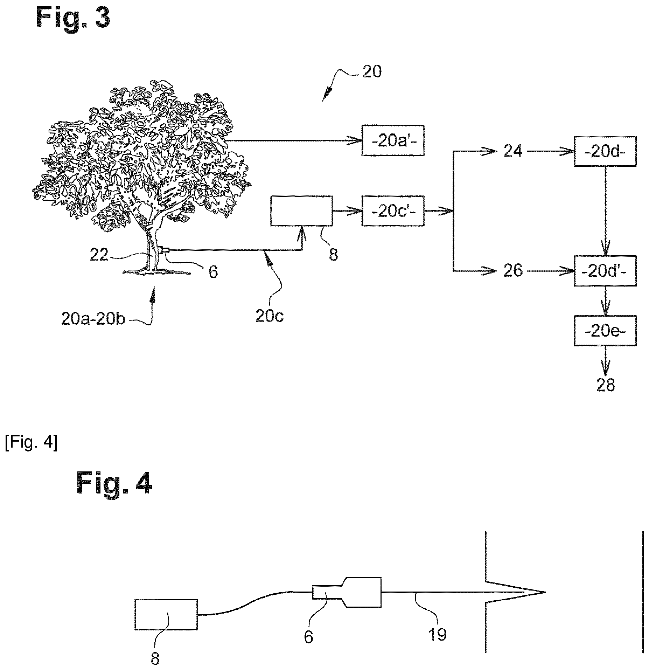

Dans une quatrième étape 140, l'espèce à laquelle appartient l'arbre est identifiée à partir du spectre infrarouge obtenu à l'étape 3 et de spectres proche infrarouge de référence correspondant à des espèces connues. Selon un mode de réalisation préféré, la détermination de l'espèce de l'arbre une fois le spectre infrarouge obtenu est réalisée au moyen d'un étalonnage préalablement mis au point. Selon ce mode de réalisation préféré, l'outil de discrimination est mis au point par une méthode 20 de régression des moindres carrés partiels comme illustré sur la figure 3.

Un arbre 22 est tout d'abord analysé dans une étape 20a', par exemple par analyse génétique d'un prélèvement de l'arbre, pour connaître son espèce. Cette opération est également réalisée pour une pluralité d'arbres.

La méthode 20 comporte une étape 20a dans laquelle un opérateur applique sur un arbre vivant 22 de référence dont l'espèce est connue un rayonnement infrarouge incident au moyen d'un spectromètre infrarouge 6.

Dans une étape 20b, le spectromètre reçoit, pour l'arbre 22, un rayonnement réfléchi émis par des molécules organiques présentes dans cet arbre sous l'effet du rayonnement infrarouge incident.

Dans une étape 20c, le rayonnement réfléchi est traité par l'unité centrale 8 pour obtenir le spectre proche infrarouge de l'arbre 22. Les étapes 20a, 20b et 20c sont ensuite effectuées sur une pluralité d'arbres dont l'espèce est connue. Dans une étape 20c', les spectres sont traités de manière classique en appliquant au moins une méthode permettant de diminuer le bruit et d'améliorer la qualité du spectre, cette méthode étant choisie parmi la normalisation, la correction de ligne de base, le lissage, la dérivation, ou une combinaison de ces méthodes.

Suite à l'étape 20c', la pluralité d'arbres est partagée en deux groupes, à savoir un groupe de calibration 24 comportant 75% des arbres étudiés et un groupe de test 26 comportant les 25% restants des arbres étudiés. Selon d'autres modes de réalisation possibles, le pourcentage des arbres placés dans le groupe de calibration et le pourcentage des arbres placés dans le groupe de test sont variables.

Dans une étape 20d, un modèle d'étalonnage test permettant d'identifier l'espèce d'un arbre à partir de son spectre IR est construit à partir du groupe de calibration 24 et dans une étape 20d', le modèle d'étalonnage test est validé sur les arbres du groupe de test 26, ou, dans le cas où il ne fonctionne pas, optimisé, de manière à obtenir un modèle d'étalonnage final.

Dans une étape 20e, un modèle d'étalonnage 28 permettant au spectromètre de déterminer l'espèce d'un arbre à partir du spectre infrarouge dudit arbre est mis au point en utilisant le modèle d'étalonnage test ainsi que les tests effectués sur le groupe de test 26.

Le modèle d'étalonnage 28 peut alors être utilisé pour identifier l'espèce d'un arbre comme par exemple l'arbre 1.

La figure 4 illustre un mode de réalisation dans lequel la méthode selon l'invention est utilisée pour obtenir le spectre infrarouge du bois de coeur de l'arbre, encore appelé duramen.

Pour cela, un carottage est réalisé dans le tronc de l'arbre 1 puis, une fibre optique 19 reliée à un spectromètre 6 est insérée à la profondeur souhaitée du trou obtenu. La prise du spectre infrarouge est alors réalisée comme dans l'exemple de réalisation décrit à la figure 2a.

## Revendications

1. Procédé (100) d'identification d'une espèce d'arbre **caractérisé en ce qu'**il comprend les étapes suivantes :
- étape 1 (110) : appliquer contre un arbre (1) entier un rayonnement proche infrarouge incident (10) au moyen d'un spectromètre infrarouge (6),
- étape 2 (120) : recevoir, au moyen dudit spectromètre, un rayonnement réfléchi (14) émis par des molécules organiques (12) présentes dans ledit arbre (1),
- étape 3 (130) : traiter le rayonnement réfléchi (14) pour obtenir un spectre proche infrarouge correspondant audit arbre,
- étape 4 (140) : identifier à quelle espèce appartient ledit arbre entier à partir du spectre infrarouge obtenu à l'étape 3 et de spectres proche infrarouge de référence correspondant à des espèces connues.

2. Procédé selon la revendication 1, **caractérisé en ce qu'**il comporte, préalablement à l'étape 1, les étapes suivantes :
étape a : appliquer sur une pluralité d'arbres entiers de référence dont l'espèce est connue un rayonnement proche infrarouge incident au moyen d'un spectromètre proche infrarouge,
étape b : recevoir, pour chaque arbre et au moyen dudit spectromètre, un rayonnement réfléchi émis par des molécules organiques présentes dans ladite pluralité d'arbres de référence sous l'effet du rayonnement infrarouge incident,
étape c : traiter, pour chaque arbre, le rayonnement réfléchi pour obtenir une pluralité de spectres proche infrarouge de référence,
étape d : attribuer chaque spectre proche infrarouge obtenu à l'espèce connue de l'arbre correspondant, et
étape e : développer un modèle d'étalonnage permettant d'identifier l'espèce d'un arbre à partir du spectre infrarouge dudit arbre.

3. Procédé selon la revendication 1 ou 2, **caractérisé en ce qu'**il comporte une étape d'enlever une portion de l'écorce de l'arbre de manière à découvrir une surface (4) de la partie interne de l'arbre (1), et émettre le rayonnement infrarouge (10) sur ladite surface (4).

4. Procédé selon la revendication 1 ou 2, **caractérisé en ce qu'**il comprend une étape d'effectuer un carottage de manière à découvrir une zone du coeur de l'arbre, et d'émettre le rayonnement infrarouge sur ladite zone.

5. Procédé selon la revendication 1 ou 2, **caractérisé en ce qu'**il comporte une étape 3' de traitement du spectre infrarouge obtenu à l'étape 3, ladite étape 3' comportant l'application d'au moins une méthode de traitement permettant de diminuer le bruit et d'améliorer la qualité du spectre.

6. Procédé selon la revendication 5, **caractérisé en ce que** l'étape 3' comporte l'application d'une méthode choisie parmi la normalisation, la correction de ligne de base, le lissage, la dérivation, ou de leurs combinaisons.

7. Procédé selon l'une quelconque des revendications précédentes, **caractérisé en ce que** les étapes 1, 2 et 3 sont réitérées une ou plusieurs fois de manière à obtenir un ou plusieurs spectres proche infrarouge qui sont ensuite combinés pour obtenir un spectre proche infrarouge moyen, ledit spectre infrarouge moyen utilisé pour effectuer l'étape 4 de manière à identifier l'espèce de l'arbre entier.

8. Procédé selon l'une quelconque des revendications précédentes, **caractérisé en ce que** le spectromètre infrarouge utilisé est un spectromètre portable (6).

9. Procédé selon l'une quelconque des revendications précédentes, **caractérisé en ce que** l'arbre entier (1) est un chêne sessile, un chêne pédonculé, un chêne pubescent, un chêne tauzin ou une de leurs hybrides.

10. Utilisation du procédé selon l'une quelconque des revendications précédentes pour déterminer la proportion de différentes espèces d'arbres dans un peuplement forestier ou sur l'ensemble des arbres d'une coupe forestière
